# EUROPEAN PATENT SPECIFICATION

(11) **EP 1 096 882 B1**
(45) Date of publication and mention of the grant of the patent: **14.03.2007**
(21) Application number: 99936530.7
(22) Date of filing: 13.07.1999
(51) Int. Cl.: A61B 10/00

(54) **BIOPSY NEEDLE**
BIOPSIENADEL
AIGUILLE POUR BIOPSIE

(30) Priority: 15.07.1998 IT MO980157
(43) Date of publication of application: 09.05.2001
(73) Proprietor: H.S. - Hospital Service - S.P.A., 04011 Aprilia (LT) (IT)
(72) Inventor: GIUSTI, Dario, I-00125 Roma (IT)
(74) Representative: Crugnola, Pietro
(86) International application number: PCT/EP1999/004954
(87) International publication number: WO 2000/003641

(56) References cited:
- EP-A- 0 536 888
- WO-A-92/00040
- WO-A-95/24858
- WO-A-95/28876
- WO-A-97/24070
- US-A- 5 368 045

## Description

The invention concerns a biopsy needle of the guillotine type, i.e. a tool designed to take samples from organs or tissues of patients, in order to diagnose the nature of a pathological process. The biopsy needle according to the present invention is suitable, particularly, for taking samples of soft and/or compressible tissues.

From the prior art, biopsy needles of a type known as "guillotine needles" are known comprising a substantially cylindrical mandrel having an end near which a housing is provided - for example a housing obtained by flattening a portion of the mandrel - designed to receive a sample to be taken, and a hollow needle with a sharp point, said need being slidingly coupled with the outer surface of he mandrel. The housing has such dimensions as to receive a tissue sample having a size suitable for being used for histological tests.

In order to effect a biopsy, the tool is introduced into the body of a patient, keeping the mandrel drawn back into the hollow needle so that only the point of the mandrel protrudes from the needle. When the point of the mandrel reaches the area of the patient body from which a sample is to be taken, the mandrel is driven out of the needle by sliding it with respect to the needle. In this way a portion of the tissue surrounding the mandrel penetrates into the housing provided in the mandrel. Then, the housing is covered by the hollow needle so that the sharp point of the hollow needle separates from the surrounding tissue, like a guillotine, said tissue portion penetrated in the housing.

Biopsy needles are known in which the displacement of the hollow needle for covering the housing of the mandrel is obtained by using pushing means, for example elastic pushing means which may be driven by the operator.

Said elastic push means are driven by releasing a stop element of said elastic means.

Usually, the force required for releasing the stop element is very small, in order to avoid an unwanted displacement of the biopsy needle when said stop element is released. However, since only a small force is required to release the stop element, there is a risk that the operator releases the stop element fortuitously before positioning the biopsy needle in the patient body correctly, If the needle is not positioned correctly into the body of the patient, there may be the need to repeat the biopsy, which causes additional discomfort and pain for the patient.

It is known from EP-A-0 536888 a tissue sampling apparatus comprising a mandrel comprising:
- a distal end having an angled point near which a housing designed to receive a tissue sample is provided, said mandrel being mounted on a first sliding member;
- an hollow needle mounted on a second sliding member and slidably coupled with the outer surface of the mandrel ;
- first pushing means comprising a first spring for pushing said first sliding member and said mandrel in a preestablished direction;
- second pushing means comprising a second spring for pushing said second sliding member and said hollow needle in said preestablished direction;
- first retaining means capable of engaging said first sliding member for preventing it from sliding in said preestablished direction;
- first releasing means for disengaging said first retaining means from said first sliding member;
- second retaining means capable of engaging said second sliding member for preventing it from sliding in said preestablished direction;
- second releasing means for disengaging said second retaining means from said second sliding member;
- locking means for locking said first releasing means in order to prevent it from being unadvertently actuated.

It is an object of the present invention to eliminate the disadvantages mentioned above.

According to the present invention, a biopsy needle is provided comprising a substantially cylindrical mandrel, having a point near which a housing is provided designed to receive a sample of tissue to be taken, a hollow needle with a sharp point, slidingly coupled with the outer surface of said mandrel, pushing means capable of causing said hollow needle to slide on said mandrel, releasable stop means of said pushing means, releasing means of said stop means, characterized in that it further comprises locking means of said releasing means. Further advantages and characteristics of the present invention will result from the description that follows, said description being only illustrative and non-limiting and being referred to the enclosed drawings, in which:
Figure 1 is a longitudinal section of a biopsy needle according to the invention;
Figure 2 is a longitudinal section of the biopsy needle of Figure 1, taken through a plane perpendicular to the section plane of Figure 1;
Figure 3 is a section like that of Figure 1, showing a first operative position of the biopsy needle;
Figure 4 is an enlarged detail of Figure 3;
Figure 5 is a section like that of Figure 3, showing a second operative position of the biopsy needle;
Figure 6 is an enlarged detail of Figure 5;

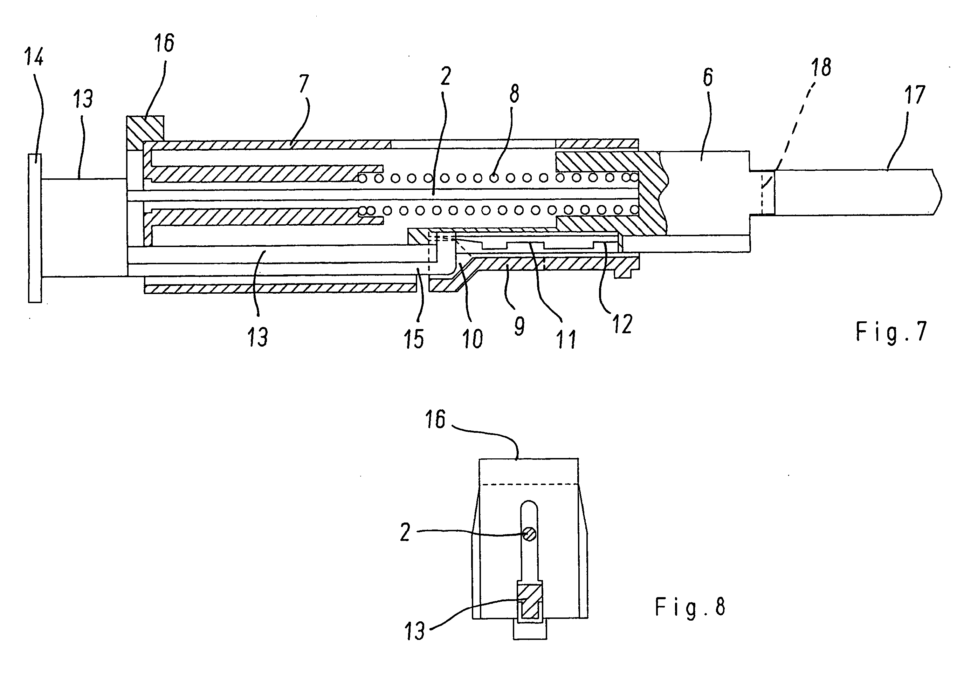

Figure 7 is an enlarged detail of Figure 1;
Figure 8 is the section VIII-VIII of Figure 1, showing in detail the locking means according to the invention;
Figure 9 is a longitudinal partial and interrupted section of a second version of a biopsy needle according to the invention;
Figure 10 is the section X-X of the Figure 9;
Figure 11 is a section like that of Figure 9, showing a third version of a biopsy needle according to the invention;

With reference to the figures, the biopsy needle 1 according to the invention comprises piercing mandrel means 2, terminating at a distal end with a point 3, designed to make easier the penetration of the mandrel means 2 through the tissues of the patient body, and a needle hollow body 4, sliding on the mandrel 2. and having a sharp end 31 turned towards the point 3 of the mandrel 2.

On the outer surface of the mandrel 2, near the point 3, a housing 5 is provided designed to receive the tissue sample to be taken for the biopsy, said housing having dimensions suitable for receiving a tissue sample of sufficient size for the histological tests to be performed on it.

The mandrel 2 is fixed, at an intermediate portion thereof, to first support means 6 slidable inside driving means 7, having, for example, the shape of a box-shaped case. The sliding of support means 6 inside the driving means 7 is opposed by elastic means 8, for example a helical spring. The driving means 7 is equipped with an elastic pawl 9 provided with a pair of stop teeth 10 capable of engaging seats 11, 12 provided in said first support means 6 for locking said first support means 6 in preestablished positions, for example in a first preestablished position and in a second preestablished position.

A proximal end of the mandrel 2, opposite to the point 3, is fixed to second support means 13 slidingly coupled to said driving means 7 and equipped with coupling means 15 capable of removably coupling with said support means 6, and releasing the stop teeth 10 of the elastic pawl 9 from the seats 11, 12 of the first support means.

The second support means 13 is provided with a handle 14 allowing an operator to slide the second support means 13 in a direction parallel to the longitudinal axis of the mandrel 2.

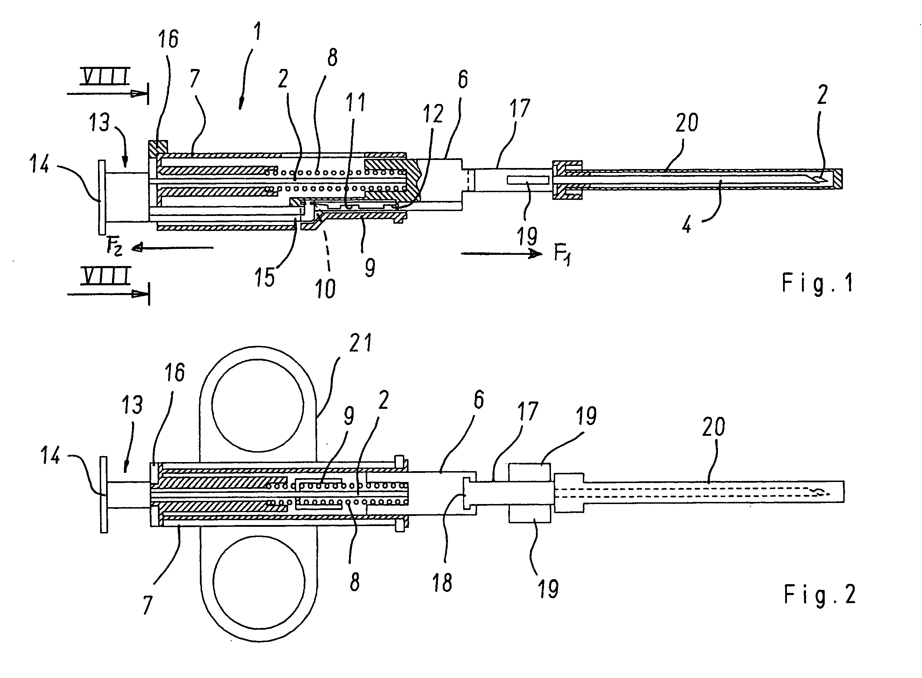

The second support means 13 is connected to locking means 16 capable of stopping said second support means 13 from sliding in the direction of arrow F1 (Figure 1) with respect to the driving means 7, in order to prevent the coupling means 15 from releasing the teeth 10 of the pawl 9 from the seats 11, 12 of the first support means 6. The locking means 16 may comprise a spacer, that can be removably interposed between the second support means 13 and the driving means 7.

The needle hollow body 4 is fixed to a support element 17 removably connected to said first support means 6 by means of a bayonet-joint 18. The support element 17 is provided with control wings 19 allowing an operator to release the needle hollow body 4 from the support element 17 and to drive it out of the mandrel 2. The mandrel 2 and the needle hollow body 4 may be isolated from the outer environment, before being used, by means of protection means 20, having for instance the shape of a cylindrical sheath. The driving means 7 may be provided with a handle 21 in order to make easier for an operator to handle the biopsy needle.

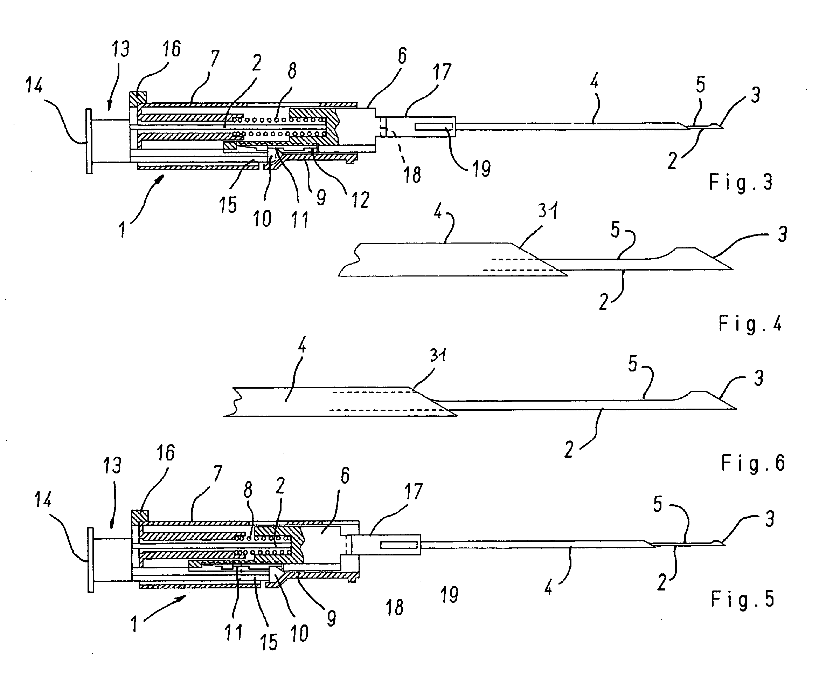

The operation of the biopsy needle 1 according to the invention takes place in the following manner: the operator, after locating the region of the patient body from which a sample to be analyzed is to be taken, acts on the handle 14 of the second support means 13 causing the second support means 13 to slide, together with the mandrel 2, in the direction of the arrow F2 (Figure 1). While the second support means 13 and the mandrel 2 are sliding, the coupling means 15 links the second support means 13 and the first support means 6 together, causing the first support means 6 and the needle hollow body 4 to slide together with the second support means 13 until the teeth 10 of the elastic pawl 9 engage the seats 11, or 12, of the first support means 6, locking said first support means 6 in a first preestablished position, or in a second preestablished position, respectively. The sliding of the first support means 6 in the direction of the arrow F2 causes a compression of the spring 8. After the first support means 6 has been locked in said first preestablished position, or in said second preestablished position, the needle hollow body 4 protrudes over the mandrel 2 leaving only the point 3 thereof free and covering the housing 5 completely (Figure 1 and 2). Now, the operator introduces the biopsy needle 1 into the patient body, until the point 3 of the mandrel 2 reaches the region from which a sample to be analyzed is to be taken. One the point 3 of the mandrel 2 has reached said region, the operator slides the second support means 13 in the direction of the arrow F1, until the spacer stops the sliding of the second support means 13, before the coupling means 15 releases the teeth 10 of the pawl 9 from the seat 11, or 12. The sliding of the second support means 13 in the direction of the arrow F1 causes the mandrel 2 to come out of the needle hollow body 4 through a distance depending on the position at which the first support means 6 has been locked. For example, if the first support means 6 has been locked in said first preestablished position, the mandrel 2 comes out of the needle hollow body 4 uncovering only in part the housing 5 designed to receive the sample to be taken (Figure 3 and 4). On the contrary, if the first support means 6 has been locked in said second preestablished position, the mandrel 2 comes out of the needle hollow body 4 until the housing 5 is completely uncovered (Figure 5 and 6). The choice of the preestablished position at which the first support means 6 is to be locked depends on the dimensions of the tissue sample that is to be taken. It is possible to preestablish more than two positions for locking at which the first support means 6 may be locked, in order to allow the biopsy needle 1 to take tissue samples of different dimensions.

When the mandrel 2 comes out of the needle hollow body 4, a portion of the surrounding tissue, that will constitute the sample to be taken, enters the housing surrounding tissue, that will constitute the sample to be taken, enters the housing 5. At this point, the operator removes the spacer and pushes again the second support means 13 in the direction of the arrow F1, until the coupling element 15, interacting with the elastic pawl 9, disengages the teeth 10 from the seat 11, or 12, releasing the first support means 6 from the second support means 13 and allowing the spring 8 to push suddenly the first support means 6, and the needle hollow body 4 in the direction of the arrow F1, until the needle hollow body 4 covers the housing 5 completely, severing from the surrounding tissue, with its sharp end 31, the tissue portion penetrated into the housing slot 5 and entrapping said tissue portion inside the needle hollow body 4.

In order to recover the tissue sample after the biopsy needle 1 has been drawn out from the body of the patient, the needle hollow body 4 is released from the first support means 6 by rotating it by means of the wings 19, until the bayonet-joint 18 is disengaged from the first support means 6, and is drawn out from the mandrel 2, uncovering the housing 5 containing the tissue sample.

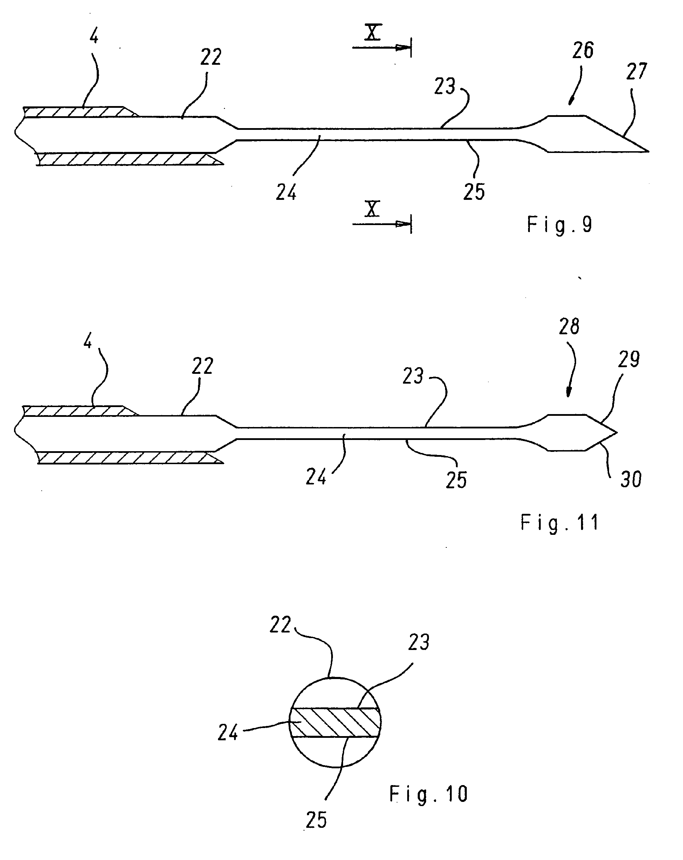

In Figures 9 and 10 a second version of a biopsy needle according to an embodiment of the invention is shown, in which the mandrel 22 is provided with two oppositely disposed housings 23, 25 arranged symmetrically with respect to a longitudinal axis of the mandrel 22 and separated by a central region 24 of mandrel, said oppositely disposed housings 23, 25 being designed to receive two tissue samples to be analyzed. This makes possible to take two tissue samples to be analyzed in a single operation, allowing for a great reduction of patient suffering and time saving.

The mandrel 22 terminates at a distal end with a point 26 provided with a flattened portion 27, forming a preestablished angle with respect to the longitudinal axis of the mandrel 22, said flattened portion 27 giving a so-called "flute nozzle" shape to the point 26, said shape making easier for the point 26 to penetrate into the tissues of the body of the patient.

In Figure 11 a third version of a biopsy needle according to the invention is shown, in which the mandrel 22 is provided, as in the second version previously described, with two oppositely disposed housings 23, 25 separated by a central region 24 of the spindle. In this third version, the mandrel 22 terminates at a distal end with a point 28, provided with two flattened portions 29, 30 angled symmetrically with respect to the longitudinal axis of the mandrel 22, said flattened portions 29, 30 giving a symmetrical wedge-shaped configuration to the point 28, which is suitable for minimizing lateral bending of the mandrel, when the mandrel, when the mandrel is introduced into the body of the patient.

## Claims

1. Biopsy needle (1) comprising mandrel means (2; 22) a distal end of which is provided with a point (3; 26; 28) near which at least one housing (5; 23; 25) is provided designed to receive a sample of tissue to be taken, hollow needle means (4) slidingly coupled with the outer surface of said mandrel means (2), pushing means (6, 8) capable of causing said hollow needle means (4) to slide on said mandrel means (2) in a preestablished direction (F1), stop means (9, 10) capable of engaging said pushing means (6, 8) in order to prevent said hollow needle means (4) from sliding in said preestablished direction (F1), releasing means (14, 15) capable of disengaging said stop means (9, 10) from said pushing means (6, 8), **characterized in that** said releasing means (14, 15) comprises locking means (16).

2. Biopsy needle according to the claim 1, wherein said hollow needle means (4) is provided with a sharp end (31) at a distal end of said hollow needle means (4) turned towards said point (3; 26; 28) of said mandrel means (2), .

3. Biopsy needle according to the claim 1, or 2, wherein a proximal end of said hollow needle means (4), opposed to said distal end, is removably coupled with first support means (6) slidably coupled with driving means (7).

4. Biopsy needle according to any of preceding claims, wherein said distal end of said hollow needle means (4) is fixed to a support element (17) coupled with said first support means (6) through bayonet-joint means (18).

5. Biopsy needle according to claim 4, wherein said support element (17) is provided with control means (19).

6. Biopsy needle according to any of claims 3 to 5, wherein said driving means (7) are shaped as a box-shaped case.

7. Biopsy needle according to any of claims 3 to 6, wherein said mandrel means (2) is fixed, at an intermediate portion thereof, to said first support means (6)

8. Biopsy needle according to any of preceding claims, wherein said pushing means (6, 8) comprises elastic means (8) connected to said first support means (6).

9. Biopsy needle according to claim 8, wherein said elastic means (8) is capable of exerting onto said first support means (6) a thrust directed towards said point (3; 26; 28) of said mandrel means (2).

10. Biopsy needle according to claim 8, or 9, when depending from claim 6, wherein said elastic means (8) is inserted inside said driving means (7).

11. Biopsy needle according to any of claims 3 to 10, wherein a proximal end of said mandrel means (2) opposed to said point (3; 26; 28) is fixed to second support means (13) slidingly to said driving means (7).

12. Biopsy needle according to claim 11, wherein said second support means (13) is provided with coupling means (15) capable of linking said second support means (13) to said first support means (6).

13. Biopsy needle according to any of preceding claims, wherein said stop means (9, 10) comprises elastic pawl means (9) connected to said driving means (7) and provided with at least one stop tooth (10) capable of engaging said first support means (6).

14. Biopsy needle according to claim 13, wherein said first support means (6) is provided with at least one seat (11, 12) which may be engaged by said at least one stop tooth (10).

15. Biopsy needle according to any of claims 12 to 14, wherein said coupling means (15) is capable of interacting with said elastic pawl means (9) in order to disengage said elastic pawl means (9) from said first support means (6).

16. Biopsy needle according to any of preceding claims, wherein said locking means comprises removable spacer means (16) capable of preventing said coupling means (15) from interacting with said elastic pawl means (9).

17. Biopsy needle (1) according to any preceding claims, wherein said at least one housing (5; 23, 25) comprises at least two housings (23, 25) separated from each other.

18. Biopsy needle according to claim 17, wherein said at least two housings (23, 25) are symmetrically arranged with respect to a longitudinal axis of said mandrel means (22).

19. Biopsy needle according to claim 18, wherein said at least two housings (23, 25) are separated by a central region (24) of said mandrel means (22).

20. Biopsy needle according to any of claims 17 to 19, wherein said point (26) is provided with a flattened portion (27) angled with respect to a longitudinal axis of the mandrel means (22).

21. Biopsy needle according to any of claims 17 to 19, wherein said point (28) is provided with two flattened portions (29, 30) angled substantially symmetrically with respect to a longitudinal axis of said mandrel means (22).

## Patentansprüche

1. Biopsienadel (1) mit Mandrin-Mitteln (2; 22), deren distales Ende einen Punkt (3; 26; 28) aufweist, in dessen Nähe zumindest ein Gehäuse (5; 23; 25) zur Aufnahme einer zu entnehmenden Gewebeprobe vorgesehen ist, ferner mit Hohlnadel-Mitteln (4), die mit der äußeren Oberfläche der Mandrin-Mittel (2) gleitend verbunden sind, mit Schiebemitteln (6, 8), durch welche die Hohlnadel-Mittel (4) auf den Mandrin-Mitteln (2) in eine vorbestimmte Richtung (F1) gleiten können, mit Stoppmitteln (9, 10), die in die Schiebemittel (6, 8) eingreifen können, um das Gleiten der Hohlnadel-Mittel (4) in die vorbestimmte Richtung (F1) zu verhindern, ferner mit Freisetzungsmitteln (14, 15), mit welchen die Stoppmittel (9, 10) von den Schiebemitteln (6, 8) gelöst werden können, **dadurch gekennzeichnet, dass** die Freisetzungsmittel (14, 15) Verschlussmittel (16) aufweisen.

2. Biopsienadel nach Anspruch 1, bei welcher die Hohlnadel-Mittel (4) mit einem scharfen Ende (31) an einem distalen Ende der Hohlnadel-Mittel (4) ausgestattet sind, das in Richtung des Punktes (3; 26; 28) der Mandrin-Mittel (2) gerichtet ist.

3. Biopsienadel nach Anspruch 1 oder 2, bei welcher ein proximales Ende der Hohlnadel-Mittel (4), das dem distalen Ende entgegengesetzt liegt, mit ersten Trägermitteln (6) lösbar verbunden ist, welche mit Antriebsmitteln (7) gleitend verbunden sind.

4. Biopsienadel nach einem der vorstehenden Ansprüche, bei welcher das distale Ende der Hohlnadel-Mittel (4) an ein Trägerelement (17) befestigt ist, welches mit den ersten Trägermitteln (6) über Bajonettverbindungsmittel (18) verbunden ist.

5. Biopsienadel nach Anspruch 4, bei welcher das Trägerelement (17) mit Steuerungsmitteln (19) ausgestattet ist.

6. Biopsienadel nach einem der Ansprüche 3 bis 5, bei welcher die Antriebsmittel (7) als eine schachtelförmige Hülle ausgebildet sind.

7. Biopsienadel nach einem der Ansprüche 3 bis 6, bei welcher die Mandrin-Mittel (2) über einen Zwischenabschnitt an die ersten Trägermittel (6) fixiert sind.

8. Biopsienadel nach einem der vorstehenden Ansprüche, bei welcher die Schiebemittel (6, 8) elastische Mittel (8) aufweisen, die mit den ersten Trägermitteln (6) verbunden sind.

9. Biopsienadel nach Anspruch 8, bei welchem die elastischen Mittel (8) auf die ersten Trägermittel (6) einen Druck ausüben können, der in Richtung des Punktes (3; 26; 28) der Mandrin-Mittel (2) gerichtet ist.

10. Biopsienadel nach Anspruch 8 oder 9, wenn diese von Anspruch 6 abhängen, bei welchen die elastischen Mittel (8) innerhalb der Antriebsmittel (7) eingebracht sind.

11. Biopsienadel nach einem der Ansprüche 3 bis 10, bei welcher ein proximales Ende der Mandrin-Mittel (2), das dem Punkt (3; 26; 28) gegenüberliegt, an zweite Trägermittel (13) fixiert ist, gleitend zu den Antriebsmitteln (7).

12. Biopsienadel nach Anspruch 11, bei welcher die zweiten Trägermittel (13) mit Verbindungsmitteln (15) ausgestattet sind, welche die zweiten Trägermittel (13) mit den ersten Trägermitteln (6) verbinden können.

13. Biopsienadel nach einem der vorstehenden Ansprüche, bei welchen die Stoppmittel (9, 10) elastische Sperrmittel (9) aufweisen, die mit den Antriebsmitteln (7) verbunden sind und die mit zumindest einem Stoppzahn (10) versehen sind, welcher in die ersten Trägermittel (6) eingreifen kann.

14. Biopsienadel nach Anspruch 13, bei welcher die ersten Trägermittel (6) mit zumindest einer Aufnahme (11, 12) ausgestattet sind, in welche der zumindest eine Stoppzahn (10) eingreifen kann.

15. Biopsienadel nach einem der Ansprüche 12 bis 14, bei welcher die Verbindungsmittel (15) mit den elastischen Sperrmitteln (9) wechselwirken können, um die elastischen Sperrmittel (9) von den ersten Trägermitteln (6) zu lösen.

16. Biopsienadel nach einem der vorstehenden Ansprüche, bei welcher die Verschlussmittel entfernbare Abstandshaltermittel (16) aufweisen, mit welchen die Verbindungsmittel (15) daran gehindert werden können, mit den elastischen Sperrmitteln (9) in Wechselwirkung zu treten.

17. Biopsienadel (1) nach einem der vorstehenden Ansprüche, bei welcher das zumindest eine Gehäuse (5; 23, 25) mindestens zwei Gehäuse (23, 25) aufweist, die voneinander getrennt sind.

18. Biopsienadel nach Anspruch 17, bei welcher die zumindest zwei Gehäuse (23, 25) bezüglich einer Längsachse der Mandrin-Mittel (22) symmetrisch angeordnet sind.

19. Biopsienadel nach Anspruch 18, bei welcher die zumindest zwei Gehäuse (23, 25) durch einen zentralen Abschnitt (24) der Mandrin-Mittel (22) getrennt sind.

20. Biopsienadel nach einem der Ansprüche 17 bis 19, bei welcher der Punkt (26) mit einem abgeflachten Abschnitt (27) versehen ist, der bezüglich einer Längsachse der Mandrin-Mittel (22) abgewinkelt ist.

21. Biopsienadel nach einem der Ansprüche 17 bis 19, bei welcher der Punkt (28) mit zwei abgeflachten Abschnitten (29, 30) versehen ist, die bezüglich einer Längsachse der Mandrin-Mittel (22) im Wesentlichen symmetrisch abgewinkelt sind.

## Revendications

1. Aiguille (1) à biopsie, comprenant des moyens de mandrin (2 ; 22) dont une extrémité distale est munie d'une pointe (3 ; 26 ; 28) près de laquelle se trouve au moins un logement (5 ; 23 ; 25) conçu pour recevoir un échantillon du tissu à prélever, des moyens d'aiguille creuse (4) couplés de manière coulissante à la surface extérieure desdits moyens de mandrin (2), des moyens de poussée (6, 8) aptes à obliger lesdits moyens d'aiguille creuse (4) à glisser sur lesdits moyens de mandrin (2) dans une direction prédéfinie (F1), des moyens d'arrêt (9, 10) aptes à engager lesdits moyens de poussée (6, 8) de manière à empêcher lesdits moyens d'aiguille creuse (4) à coulisser dans ladite direction prédéfinie (F1), des moyens de libération (14, 15) aptes à désengager lesdits moyens d'arrêt (9, 10) desdits moyens de poussée (6, 8), ***caractérisée en ce que*** lesdits moyens de libération (14, 15) comprennent des moyens de verrouillage (16).

2. Aiguille à biopsie selon la revendication 1, dans laquelle lesdits moyens d'aiguille creuse (4) sont munis d'une extrémité pointue (31) sur une extrémité distale desdits moyens d'aiguille creuse (4) orientée vers ladite pointe (3 ; 26 ; 28) desdits moyens de mandrin (2).

3. Aiguille à biopsie selon la revendication 1 ou 2, dans laquelle une extrémité proximale desdits moyens d'aiguille creuse (4) opposée à ladite extrémité distale, est couplée de manière amovible à des premiers moyens de support (6) couplés de manière coulissante à des moyens d'entraînement (7).

4. Aiguille à biopsie selon l'une quelconque des revendications précédentes, dans laquelle ladite extrémité distale desdits moyens d'aiguille creuse (4) est fixée à un élément de support (17) couplé auxdits premiers moyens de support (6) par des moyens de joint à baïonnette (18).

5. Aiguille à biopsie selon la revendication 4, dans laquelle ledit élément de support (17) est muni de moyens de commande (19).

6. Aiguille à biopsie selon l'une quelconque des revendications 3 à 5, dans laquelle lesdits moyens d'entraînement (7) adoptent la forme d'un boîtier.

7. Aiguille à biopsie selon l'une quelconque des revendications 3 à 6, dans laquelle lesdits moyens de mandrin (2) sont fixés, au niveau d'une portion intermédiaire, auxdits premiers moyens de support (6).

8. Aiguille à biopsie selon l'une quelconque des revendications précédentes, dans laquelle lesdits moyens de poussée (6, 8) comprennent des moyens élastiques (8) reliés auxdits premiers moyens de support (6).

9. Aiguille à biopsie selon la revendication 8, dans laquelle lesdits moyens élastiques (8) sont capables d'exercer sur lesdits premiers moyens de support (6) une poussée dirigée vers ladite pointe (3 ; 26 ; 28) desdits moyens de mandrin (2).

10. Aiguille à biopsie selon la revendication 8 ou 9, lorsqu'elles sont rattachées à la revendication 6, dans laquelle lesdits moyens élastiques (8) sont insérés à l'intérieur desdits moyens d'entraînement (7).

11. Aiguille à biopsie selon l'une quelconque des revendications 3 à 10, dans laquelle une extrémité proximale desdits moyens de mandrin (2) opposée à ladite pointe (3 ; 26 ; 28) est fixée à des deuxièmes moyens de support (13) de manière coulissante par rapport auxdits moyens d'entraînement (7).

12. Aiguille à biopsie selon la revendication 11, dans laquelle lesdits deuxièmes moyens de support (13) sont munis de moyens de couplage (15) aptes à relier lesdits deuxièmes moyens de support (13) avec lesdits premiers moyens de support (6).

13. Aiguille à biopsie selon l'une quelconque des revendications précédentes, dans laquelle lesdits moyens d'arrêt (9, 10) comprennent des moyens de cliquet élastique (9) raccordés auxdits moyens d'entraînement (7) et munis d'au moins une dent d'arrêt (10) apte à engager lesdits premiers moyens de support (6).

14. Aiguille à biopsie selon la revendication 13, dans laquelle lesdits premiers moyens de support (6) sont munis d'au moins un siège (11, 12) qui peut être engagé par ladite au moins une dent d'arrêt (10).

15. Aiguille à biopsie selon l'une quelconque des revendications 12 à 14, dans laquelle lesdits moyens de couplage (15) sont aptes à interagir avec lesdits moyens de cliquet élastique (9) de manière à dégager lesdits moyens de cliquet élastique (9) desdits premiers moyens de support (6).

16. Aiguille à biopsie selon l'une quelconque des revendications précédentes, dans laquelle lesdits moyens de verrouillage comprennent des moyens d'écartement amovibles (16) apte à empêcher lesdits moyens de couplage (15) d'interagir avec lesdits moyens de cliquet élastique (9).

17. Aiguille (1) à biopsie selon l'une quelconque des revendications précédentes, dans laquelle ledit au moins un logement (5 ; 23, 25) comprend au moins deux logements (23, 25) séparés l'un de l'autre.

18. Aiguille à biopsie selon la revendication 17, dans laquelle lesdits au moins deux logements (23, 25) sont disposés symétriquement par rapport à un axe longitudinal desdits moyens de mandrin (22).

19. Aiguille à biopsie selon la revendication 18, dans laquelle lesdits au moins deux logements (23, 25) sont séparés par une région centrale (24) desdits moyens de mandrin (22).

20. Aiguille à biopsie selon l'une quelconque des revendications 17 à 19, dans laquelle ladite pointe (26) est munie d'une portion aplatie (27) formant un angle par rapport à un axe longitudinal des moyens de mandrin (22).

21. Aiguille à biopsie selon l'une quelconque des revendications 17 à 19, dans laquelle ladite pointe (28) est munie de deux portions aplaties (29, 30) formant des angles sensiblement symétriques par rapport à un axe longitudinal desdits moyens de mandrin (22).
